# EUROPEAN PATENT APPLICATION

(11) **EP 2 992 926 A1**
(43) Date of publication of application: **09.03.2016**
(21) Application number: 15189953.1
(22) Date of filing: 27.01.2003
(51) Int. Cl.: A61N 1/05, A61N 1/08

(54) **METHOD AND APPARATUS FOR SHIELDING AGAINST MRI DISTURBANCES**

(30) Priority: 29.01.2002 US 59509
(62) Divisional of application: 09012993.3
(71) Applicant: Medtronic, Inc., Minneapolis, Minnesota 55432 (US)
(72) Inventor: ZEIJEMAKER, Volkert, A., 6371 VW Landgraaf (NL)
(74) Representative: Iceton, Greg James

(57) **Abstract**

An implantable medical lead, comprising an electrical conductor 42; a braided shield 67 that surrounds the conductor 42; an insulative material 44 present at least between the shield 67 and the conductor 42; and an electrode 46 electrically coupled to the electrical conductor 42.

## Description

This invention relates generally to apparatus for electrically stimulating a heart, and, more particularly, to a method and apparatus for reducing the effects of an electro-magnetic field on the operation and safety of implantable medical devices.

Since their earliest inception some forty years ago, there has been a significant advancement in body-implantable electronic medical devices. Today, these implantable devices include therapeutic and diagnostic devices, such as pacemakers, cardioverters, defibrillators, neural stimulators, drug administering devices, among others for alleviating the adverse effects of various health ailments. Today's implantable medical devices are also vastly more sophisticated and complex than their predecessors, and are therefore capable of performing considerably more complex tasks for reducing the effects of these health ailments.

A variety of different implantable medical devices (IMD) are available for therapeutic stimulation of the heart and are well known in the art. For example, implantable cardioverter-defibrillators (ICDs) are used to treat patients suffering from ventricular fibrillation, a chaotic heart rhythm that can quickly result in death if not corrected. In operation, the ICD continuously monitors the electrical activity of a patient's heart, detects ventricular fibrillation, and in response to that detection, delivers appropriate shocks to restore normal heart rhythm. Similarly, an automatic implantable defibrillator (AID) is available for therapeutic stimulation of the heart. In operation, an AID device detects ventricular fibrillation and delivers a non-synchronous high-voltage pulse to the heart through widely spaced electrodes located outside of the heart, thus mimicking transthoracic defibrillation. Yet another example of a prior art cardioverter includes the pacemaker/cardioverter/defibrillator (PCD) disclosed, for example, in U.S. Patent No. 4,375,817 to Engle, et al. This device detects the onset of tachyarrhythmia and includes means to monitor or detect progression of the tachyarrhythmia so that progressively greater energy levels may be applied to the heart to interrupt a ventricular tachycardia or fibrillation. Numerous other, similar implantable medical devices are available, including programmable pacemakers, nerve stimulation systems, physiological monitoring devices, and the like

Modem electrical therapeutic and/or diagnostic devices for the heart and other areas of the body generally include an electrical connection between the device and the body. This connection is usually provided by a medical electrical lead. Such a lead normally takes the form of a long, generally straight, flexible, insulated conductor. At its proximal end, the lead is typically connected to a connector of the electrical therapeutic and diagnostic device, which may be implanted within the patient's body. Generally an electrode is located at or near the distal end of the lead and is attached to, or otherwise comes in contact with, the body. In the case of an implantable pacing or defibrillation system, leads are electrically coupled to tissue within the heart or within a coronary vessel. In the case of cardiac applications, a tip electrode is may be anchored to the heart tissue by means of a screw-in lead tip that can be inserted into the heart tissue. Another fixation mechanism utilizes tines that are affixed to the trebeculae of the heart. This provides a physical connection of the lead to the heart tissue. Alternatively, when an electrode is positioned within a vessel, the electrode can be shaped so that it may be wedged between the walls of the vessel. Other anchoring means are known in the art. In any of these embodiments, the area of tissue making contact with the electrode is relatively small.

Problems may be associated with implanted leads when a patient comes in contact with alternating electromagnetic fields. Such fields can induce an electric current within a conductor of the lead. In fact, in the presence of electromagnetic fields, an implanted electrical lead acts as an antenna, resulting in an electrical current that flows from the lead and through body tissue. Because the tissue area associated with electrode contact may be very small, the current densities may be high, resulting in tissue heating that can cause damage. In some instances, a high-density current of this nature may be fatal.

In addition to the risk of burning discussed above, there are other risks associated with exposure to electromagnetic fields and/or radio-frequency energy. A sudden burst of radio-frequency energy can cause an electric pulse within the lead that could send the heart into fibrillation. The implanted medical device can sense the imposed voltage on the lead and react inappropriately, resulting in the wrong therapy being administered. Examples of inappropriate therapy modification include changing the rate or thresholds associated with pacing pulses.

Alternating electrode fields are often used in medical diagnosis techniques such as Magnetic Resonance Imaging (MRI), which is a technique for producing images of soft tissue within the human body. MRI scanners from many different sources are now well known and commercially available. Magnetic resonance spectroscopic imaging (MRSI) systems are also known and are herein intended to be included within the terminology "MRI" system or scanner. These techniques can give valuable diagnostic information without the need for invasive surgery, but also subject the patient to significant alternating electromagnetic fields, resulting in the risks described above.

There is therefore a need for improved methods and apparatus that reduce the detrimental effects that are possible when a patient with an implantable electrical lead is subjected to an electromagnetic field.

### SUMMARY OF THE INVENTION

The current invention relates to a medical electrical lead comprising a lead body and an electrically-conductive shield adjacent to at least a portion of the lead body. The shield is adapted to dissipate energy when the lead is subjected to an electromagnetic field. The shield may be further coupled to a housing of an implantable medical device to enhance energy dissipation capabilities. In another embodiment, a method comprises receiving electromagnetic energy within a shield coating surrounding a portion of the lead. The energy is then dissipated in a manner that protects tissue surrounding the lead from injury.

Yet another aspect of the present invention relates to a method of manufacturing a shielded medical lead. The method comprises providing a lead having at least one electrode wire surrounded by a layer of insulative material. A shield coating capable of receiving electromagnetic energy and conducting an electrical current is applied over at least a portion of the insulative material. The shield coating is not in direct electrical communication with the electrode wire. The shield coating can comprise a sputtered metal applied to the exterior surface of the insulative material.

Other aspects of the invention will become apparent from the drawings and the accompanying description of preferred embodiments (given by way of example only).

### BRIEF DESCRIPTION OF THE DRAWINGS

The invention may be understood by reference to the following description taken in conjunction with the accompanying drawings, in which like reference numerals identify like elements, and in which:
Figure 1 schematically illustrates an implanted medical device within a human body as may be used in accordance with the present invention;
Figure 2 schematically illustrates an embodiment of an implanted medical device with an endovenous epicardial lead positioned adjacent the left ventricle of a heart;
Figure 3 schematically illustrates an embodiment of an implantable medical device with a tip electrode located at the distal end of the lead;
Figure 4 is a table showing various named regions of the electromagnetic spectrum along with their respective wavelength, frequency and energy ranges;
Figure 5 is a generalized drawing of an embodiment of the present invention;
Figure 6 is a generalized drawing of an embodiment of the present invention;
Figure 7 schematically illustrates an embodiment of an implantable medical device comprising features of the present invention;
Figure 8 schematically illustrates a cross sectional view of an embodiment of the present invention; and
Figures 9A-B show cross-sectional views of electrical leads of various embodiments of the invention.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed. The scope of the invention is as defined by the appended claims.

### DETAILED DESCRIPTION OF SPECIFIC EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

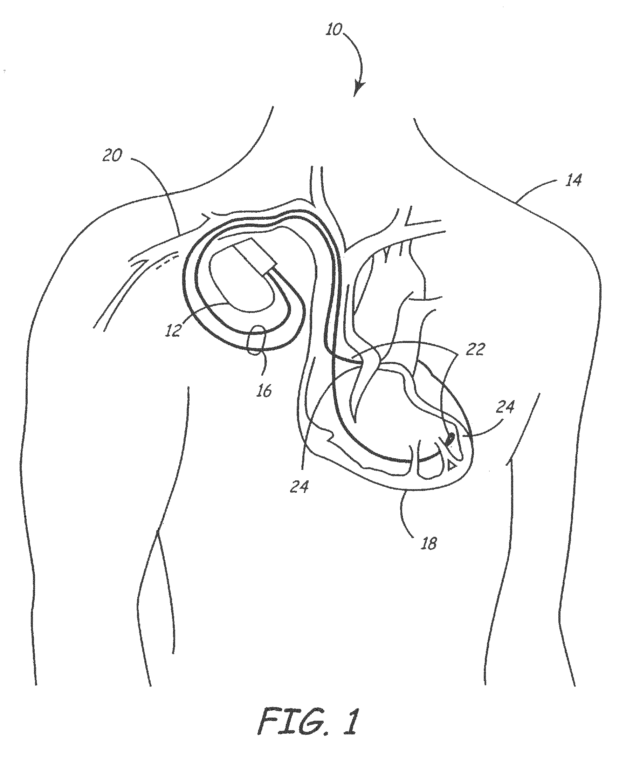

Figure 1 illustrates an implantable medical device (IMD) system 12 as may be adapted for use with the current invention. The IMD may be a pacemaker, cardioverter/defibrillator, a neurostimulation system, a hemodynamic monitor, a drug delivery device, or any other implantable device coupled to a medical electrical lead or catheter implanted in a patient's body 14. In the current example, one or more leads, collectively identified with reference numeral 16, are electrically coupled to the IMD 12, which is shown to be a pacemaker. The leads extend into the patient's heart 18 via a vein 20.

Located generally near the distal end 22 of the leads 16 are one or more exposed conductive electrodes 24 that are attached to the heart tissue for sensing cardiac activity, delivering electrical pacing stimuli, or providing a cardioversion/defibrillation shock to the heart 18. The contact area between the electrodes 24 and the heart 18 tissue may be very small as compared, for example, to the contact area between the device 12 and the body 14.

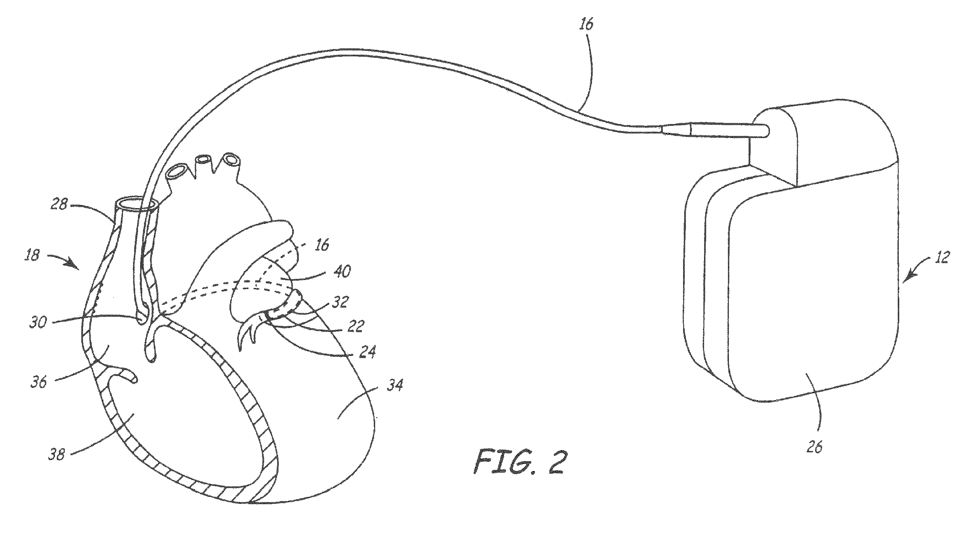

Figure 2 illustrates an implantable medical device (IMD) 12 such as a pacemaker or defibrillator as may be used with the current invention. The device 12 is housed within a hermetically sealed, biologically inert outer canister or housing 26, which may itself be conductive. One or more leads 16 are electrically coupled to the device 12 and extend to a point adjacent the patient's heart 18 via a vein, typically the superior vena cava vein 28. This embodiment illustrates a lead 16 proceeding from the right atrium 36, through the coronary sinus 30 with the distal end 22 positioned within a cardiac vein 32 that is adjacent to the left ventricle 34 of the heart 18. An electrode 24, for example, a ring electrode, is placed in contact with the cardiac vein 32 to provide electrical stimulation and/or sense signals within the heart 18. Leads can also be placed in other locations, including any of the chambers of the heart, or another location within the body as needed. This illustration shows the relatively small contact area between the electrode 24 and the body tissue.

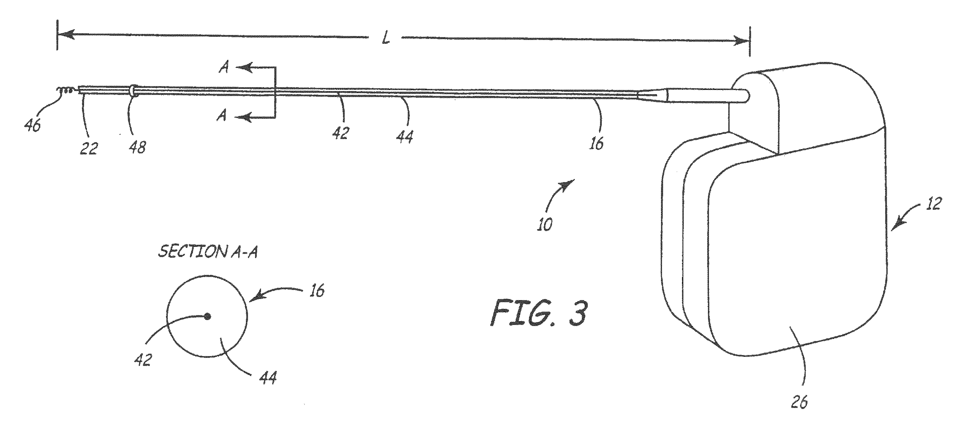

Figure 3 schematically illustrates an embodiment of an implantable medical system 10 having an electrical lead 16 having a length L. The electrical lead 16 is shown as a cross section to show the electrode wire 42 surrounded by an insulator 44. A tip electrode 46 is shown at distal end 22, and a ring electrode 48 is shown proximal tip electrode. More or fewer electrodes may be provided by the lead. One embodiment of a tip electrode 46 comprises a helical coil that can be affixed directly to heart tissue and is particularly useful in attaching the lead 16 within an atrium or ventricle chamber wall of a heart 18. The ring electrode 48 can comprise a section of the lead 16 outer surface and can comprise a protrusion, such as a ridge having a larger diameter than the remainder of the lead 16. Ring electrodes 48 are particularly useful when the lead 16 is placed within a vein, such as the cardiac vein 32 shown in Figure 2. The lead 16 can comprise more than one electrode wire 42, and in an embodiment such as shown in figure 3, separate electrode wires 42 can be connected to the tip electrode 46 and the ring electrode 48, respectively.

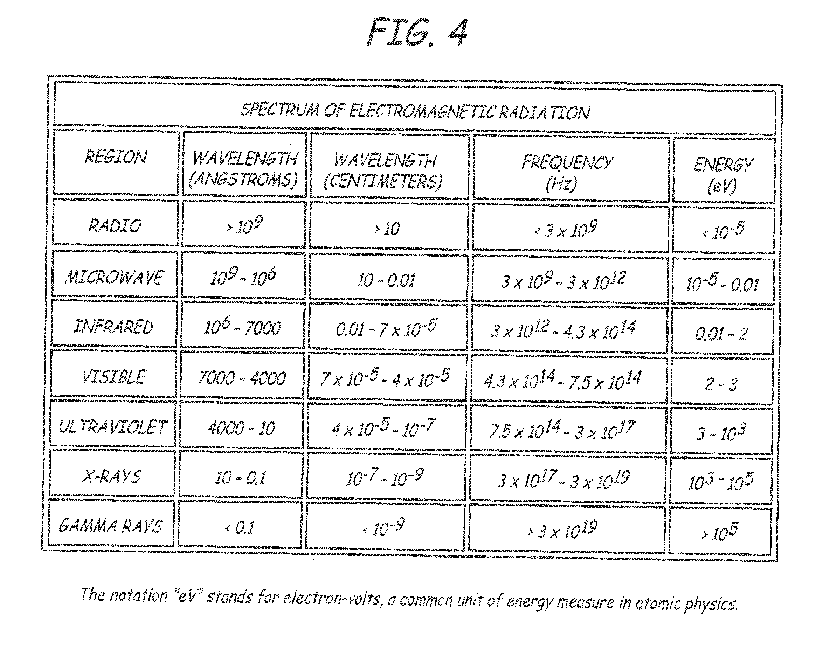

Figure 4 is a table showing various named regions of the electromagnetic spectrum along with their respective wavelength, frequency and energy ranges. As the frequency increases, the amount of energy that is transmitted increases as well. Electromagnetic fields may be described using a unit of magnetic flux density known as a "Tesla." Another unit of measure commonly used is the "gauss", where 1 Tesla equals 10,000 gauss. The magnets in use today in MRI systems generate magnetic fields having a flux density in the 0.5 - 2.0 Tesla range, or 5,000 - 20,000 gauss.
An MRI system of 1 Tesla will operate at a frequency of approximately 42 MHz or 42 x 10E6 hertz. This is within the radio region of the electromagnetic radiation spectrum and is commonly referred to as radio-frequency (RF) energy. In the search for better diagnostic capabilities, MRI systems utilizing even stronger magnets and that are capable of generating increasing amounts of RF energy are being developed. The greater the level of RF energy transmitted, the greater the risk of injuring a patient by inducing electrical currents within an implanted lead as is described below.

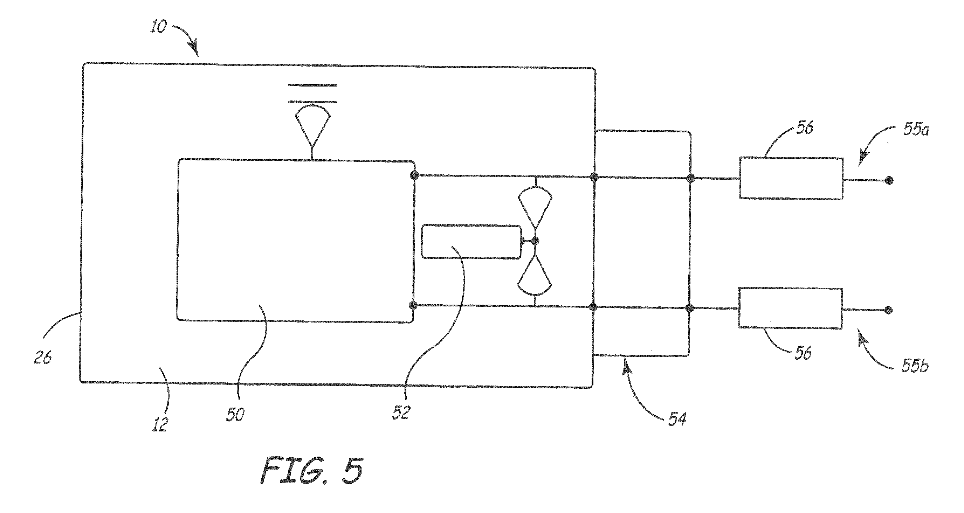

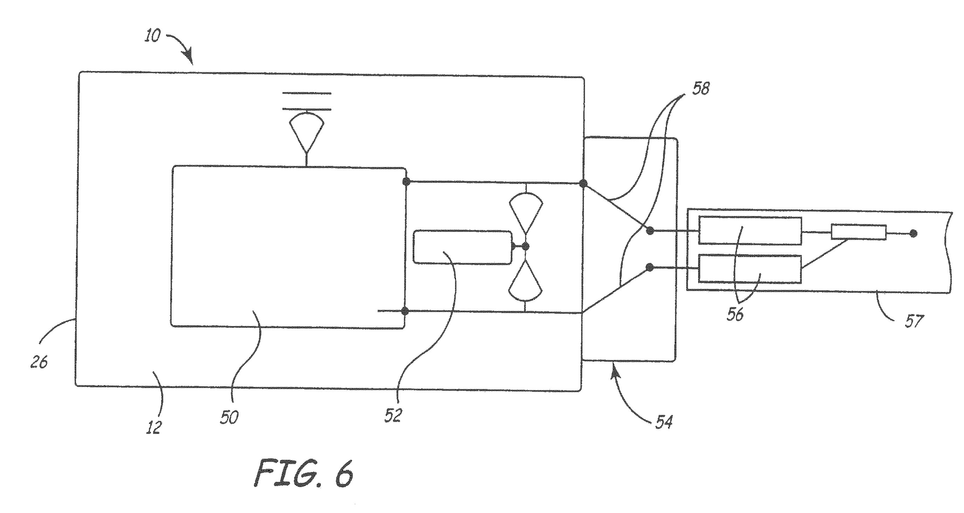

Figure 5 is a generalized drawing of an embodiment of the present invention comprising an implantable system 10 having an electronic device 12 enclosed within a housing 26. Electronics 50 and a power supply 52 are included within the device 12 and are connected to a pair of electrical leads 55a and 55b by a connector 54. These leads 16 shown in Figure 6 are referred to as unipolar leads because these leads include a single conductor. Passive or active components 56 may be incorporated within the leads 16 to decrease the effects of the magnetic field on the lead, as disclosed in EP-A-1441804. U.S. Patent Application entitled "Apparatus and Method for Shunting Induced Current in an Electrical Lead", Docket Number P-9698, filed on October 31, 2001, and a continuation-in-part of that application having the same title and filed on even date herewith, both incorporated herein by reference in its entirety.

Figure 6 is a generalized drawing of an embodiment of the present invention comprising an implantable system 10 having an electronic device 12 enclosed within a housing 26. Electronics 50 and a power supply 52 are contained within the device 12 and are connected to a pair of electrical conductors 58 that are joined within a single lead 57 by a connector 54. The single lead 57 shown in Figure 6 can be referred to as a bipolar lead because the lead includes two conductors. Passive or active components 56 may be incorporated within the electrical conductors 58 of the lead 57 to alter the effects of electromagnetic fields on the system 10 as described in EP-A-1441804 entitled "Medical Implantable Lead for Reducing Magnetic Resonance Effects" referenced above.

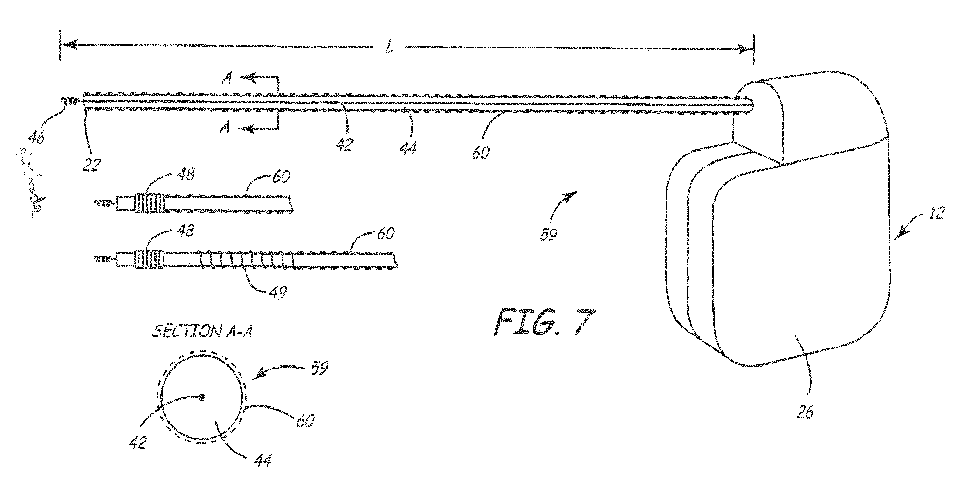

Figure 7 schematically illustrates embodiments of an implantable medical system 10 comprising features of the present invention. The electrical lead 59 is shown as a cross section to show the electrode wire 42 surrounded by an insulator 44, which may be formed of silicone, a biocompatible polymer such as polyurethane, or any other suitable biocompatible material. A tip electrode 46 is shown at its distal end 22. The particular embodiment of tip electrode 46 comprising a helical coil can be affixed directly to heart tissue and is particularly useful in attaching the lead 59 within an atrium or ventricle chamber wall of a heart 18, such as shown in Figure 1. Alternate embodiments are shown of the electrical lead 59 comprising a ring electrode 48 and/or a defibrillator coil 49. A shield coating 60 is shown on the exterior of the lead 16. The shield coating 60 is not in direct electrical contact with the electrode wire 42 or the electrode 46. The shield coating 60 can be in contact with the housing 26 of the IMD, which can act as additional surface area for dissipation of energy received by the shield coating 60 from electromagnetic waves. If desired, the shield coating may be applied only to a portion of the lead body. In this instance, the shield coating may be electrically-coupled to the housing via additional conductive traces, wires, or other conductive members adjacent or, or embedded, within the lead body.

The lead 59 must be flexible to be implanted within the body of the patient. The shield coating 60 can comprise a thin layer of material that will not substantially alter the stiffness of the lead 59. The term "not substantially alter" within the present application means that a lead with a shield coating applied retains enough flexibility to enable the lead to be implanted within the body of a patient.

The shield coating 60 can be applied to the lead 59 by numerous means, as will be appreciated by those of ordinary skill in the art upon a complete reading of this disclosure. A thin layer of the coating material in a sheet form may encircle all, or part of, the lead and be attached by fusing, gluing, or mechanically fixing the coating material onto the exterior of the lead. Alternatively, a thin layer of sputtered electrically-conductive material such as a metal may be applied on the outer surface of the lead to provide an effective shield coating 60. The sputtered material can form a shield coating 60 that has a "skin effect" on the lead 59. The skin effect acts to keep the higher frequency radio waves on the outer shield coating 60 of the lead 59 and reduce the energy transferred to the electrode wire 42. By reducing the energy transmitted to the electrode wire 42, the quantity of current that passes through the electrode 46 into the heart tissue is reduced, reducing the risk of heart tissue damage due to heating of the localized contact area between the electrode 46 and the heart.

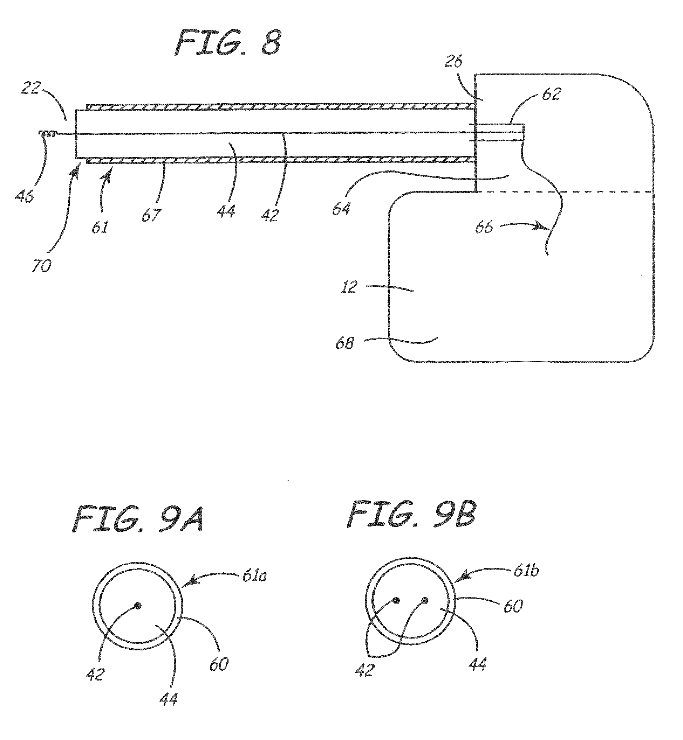

Figure 8 schematically illustrates a cross sectional view of an embodiment of the present invention comprising an implantable medical system including an electrical lead 61. The electrical lead 61 comprises an electrode wire 42 surrounded by an insulated material 44. A tip electrode 46 is shown at the distal end 22 of the lead 61. A shield 67 is located adjacent, or proximate to, at least a portion of the outer surface of the lead 61. In the illustrated embodiment, shield is a braid or other woven structure formed of conductive fibers may be placed adjacent to, or embedded within the insulative coating of the lead body. According to this embodiment, the insulated material may comprise a tubular shaft constructed of "Ultem" polyamide, or other high temperature polymer covered with a braided flat wire, another kind of suitable wire, or conductive filament and jacketed by a flexible polymer such as nylon, polyurethane, or "PEBAX"™. The shield 67 will be in direct contact with the patient's bodily tissues and fluids and therefore the fibers comprise a biocompatible conductive material. The shield 67 can be in contact with the housing 26 of the device 12, or may not extend to contact with the housing 26 so that there is no electrical path between the shield 67 and the housing 26 other than through the patient's body.

The electrode wire 42 may be connected to the electronics 50 within the device 12 by means of a connection pin 62 shown coupled to connection block 64. A conductor 66 generally provides an electrical path into the electronics section 68 of the device 12. The shield 67 is not in direct contact with the electrode wire 42, the electrode 46, or other active conductors such as electrode rings or defibrillator coils, and therefore is not in direct electrical communication with the electrical parts of the system. The only electrical path between the shield 67 and the electrode 46 is through the patient's body. The shield 67 acts as an antenna for radio-frequency waves and dissipates the energy that it receives into the surrounding blood stream or other bodily tissue or fluid. The shield 67 absorbs at least most of the radio-frequency energy that the lead 61 would otherwise be subjected to and therefore reduces the quantity of radio-frequency energy that is absorbed by the electrode wire 42. By reducing the quantity of radio-frequency energy that is absorbed by the electrode wire 42, the risk of harmful effects resulting from an induced current within the electrode wire 42 is reduced.

Figures 9A and 9B show cross-sectional views of electrical leads of various embodiments of the invention. Figure 9A shows a lead 61 a having a single conductor wire 42 surrounded by an insulating material 44. On the exterior of the insulating material 44 is a shield coating 60. Figure 9B shows a lead 61b having two conductor wires 42 that are surrounded by an electrically-insulating material 44, which is coated by a shield coating 60, as discussed above in reference to Figure 7. The thickness of the shield coating 60 can vary depending on the particular material used and other design considerations. The shield coating 60 is typically thin, so that it will not significantly restrict the flexibility or increase the diameter of the lead 16. In one embodiment of the invention the shield coating is less than two micrometers in thickness and comprises sputtered titanium, although any biologically-compatible, electrically-conductive, material having a different thickness may be used in the alternative. In either of the embodiments of Figures 9A and 9B, the shield coating may be replaced by a shield comprising a braided structure adjacent to, or embedded within, the lead body as discussed above in regards to Figure 8.

## Claims

1. An implantable medical lead, comprising:
an electrical conductor (42);
a braided shield (67) that surrounds the conductor (42);
an insulative material (44) present at least between the shield (67) and the conductor (42); and
an electrode (46) electrically coupled to the electrical conductor (42).

2. The implantable medical lead of claim 1, further comprising an electrical connection on a proximal end of the lead body, the electrical conductor (42) being electrically coupled to the electrical connection.

3. The implantable medical lead of claim 2, wherein the electrical connection comprises a pin (62).

4. The implantable medical lead of claim 1, wherein the shield (67) is embedded onto the insulative material (44).

5. The implantable medical lead of claim 1, wherein the shield (67) is adjacent to the insulative material.

6. A method of shielding an implantable medical lead, comprising:
surrounding a conductor (42) with a braided shield (67), the conductor being electrically coupled to an electrode (46); and
providing an insulative material (44) between at least the shield (67) and the conductor (42).

7. The method of claim 6, further comprising establishing an electrical connection on a proximal end of the lead body to a connection block (64) of an implantable medical device.

8. The method of claim 7, wherein the electrical connection comprises a pin (62) connected to the connection block (64).

9. The method of claim 6, wherein the shield (67) is embedded onto the insulative material (44).

10. The method of claim 6, wherein the shield (67) is adjacent to the insulative material (44).
